# EUROPÄISCHE PATENTSCHRIFT

(11) **EP 0 842 897 B1**
(45) Veröffentlichungstag und Bekanntmachung des Hinweises auf die Patenterteilung: **09.08.2000**
(21) Anmeldenummer: 97118691.1
(22) Anmeldetag: 28.10.1997
(51) Int. Cl.: C01C 3/02, C07C 253/24, C01B 17/74, C07D 213/84

(54) **Verfahren zur Herstellung von Cyanoverbindungen durch Ammonoxidation**
Process for the preparation of cyano compounds by ammonoxidation
Procédé de préparation de composés de cyano par ammonoxidation

(30) Priorität: 16.11.1996 DE 19647527
(43) Veröffentlichungstag der Anmeldung: 20.05.1998
(73) Patentinhaber: Degussa-Hüls Aktiengesellschaft, 60287 Frankfurt am Main (DE)
(72) Erfinder: von Hippel, Lukas, Dr., 63755 Alzenau (DE); Sauer, Jörg, Dr., 63517 Rodenbach (DE); Schütte, Rüdiger, Dr., 63755 Alzenau (DE); Sauer, Manfred, Dr., 63517 Rodenbach (DE); Arntz, Dietrich Dr., 61440 Oberrursel (DE)

(56) Entgegenhaltungen:
- EP-A- 0 037 123
- EP-A- 0 121 032
- EP-A- 0 311 334
- DE-C- 412 924
- US-A- 5 204 079
- CHEMICAL ABSTRACTS, vol. 92, no. 23, 9.Juni 1980 Columbus, Ohio, US; abstract no. 198128, UMEMURA, SUMIO ET AL: "2,6-Dicyanophenols" XP002058140 & JP 54 145 634 A (UBE INDUSTRIES, LTD., JAPAN)

## Beschreibung

Die Erfindung richtet sich auf ein Verfahren zur Herstellung von Cyanoverbindungen der allgemeinen Formel R-CN, worin R für H oder einen organischen Rest steht, durch Ammonoxidation in der Gasphase einer ammonoxidierbaren organischen Verbindung mit einer Quelle für Ammoniak und einer Quelle für Sauerstoff in Gegenwart eines Ammonoxidationskatalysators bei einer Temperatur im Bereich von 200 bis 700 °C.

Bei der Ammonoxidation handelt es sich um einen wohlbekannten Prozeß, wobei eine ammonoxidierbare organische Verbindung in Gegenwart eines Ammonoxidationskatalysators bei erhöhter Temperatur mit Ammoniak und Sauerstoff in eine Cyanoverbindung überführt wird. Technische Bedeutung erlangte die Ammonoxidation beispielsweise zur Herstellung von Acrylnitril aus Propen, Benzonitril aus Toluol, Phthalonitril und Terephthalonitril aus ortho- beziehungsweise para-Xylol, 3-Cyanopyridin aus 3-Picolin sowie Cyanwasserstoff aus Methan (Andrussow-Verfahren) - siehe Ullmann's Enzyclopedia of Industrial Chemistry Vol. A5, 5th ed. (1986) Seiten 328-29, 333, 343 sowie Vol. A8, 5th ed. (1987) Seiten 159-62.

Bisher wird die Ammonoxidation ausschließlich unter Einsatz von gasförmigem Ammoniak als Quelle für Ammoniak durchgeführt. Zahlreiche Katalysatoren haben sich für die Ammonoxidation als geeignet erwiesen, beispielsweise binäre Oxide auf der Basis von Bi₂O₃-MoO₃, V₂O₅-MoO₃ und V₂O₅-Sb₂O₅ zur Herstellung organischer Nitrile. Zur Herstellung von Cyanwasserstoff durch Ammonoxidation von Methanol oder Formaldehyd wird beispielhaft auf die EP-A 0 107 638 und EP-A 0 121 032, wonach Oxide von Molybdän und Eisen beziehungsweise Oxide von Phosphor und einem Element aus der Reihe Fe, Co, Ni, Zn, B und U als Katalysatoren eingesetzt werden, hingewiesen. Zahlreiche Katalysatoren zur Ammonoxidation von z. B. Propen sind der EP-A 0 311 334 zu entnehmen. Zur Ammonoxidation von Methylpyridinen und geeigneten Katalysatoren hierfür wird auf die US-Patente 4,447,612 und 4,482,719 verwiesen. Üblicherweise wird die Ammonoxidation bei einer Temperatur zwischen 200 und 600 °C durchgeführt. Eine Ausnahme stellt der Andrussow-Prozeß dar, wobei Methan bei über 1000 °C unter Verwendung von Edelmetallkatalysatoren (Pt-Rh-Netze) zu Cyanwasserstoff ammonoxidiert wird.

Aus Untersuchungen von Nozawa et.al. Mokuzai Gakkaishi, 27 (1), 49-53 (JP) (1981) (siehe Chem. Abstr. 94:105119) ist bekannt, daß bei der Pyrolyse von mit Diammoniumphosphat behandeltem Holz oder Cellulose Cyanwasserstoff gebildet wird. Mit zunehmender Temperatur nimmt die gebildete Menge zu und erreicht bei 900 °C ein Maximum von 10 ml HCN-Gas/g, entsprechend 12 mg HCN/g verbrannten Holzes. In Gegenwart von Sauerstoff beträgt die HCN-Entwicklung bei 500 bis 600 °C nur 2 bis 4 ml/g, und die HCN-Entwicklung wird bei einer Temperatursteigerung auf 700 °C in Gegenwart von Sauerstoff beträchtlich reduziert. Obgleich bei dieser Pyrolyse Cyanwasserstoff gebildet wird und Diammoniumphosphat als Quelle für Ammoniak angesehen werden kann, handelt es sich nicht um eine Ammonoxidation, da kein Katalysator anwesend ist. Die Ausbeute an Cyanwasserstoff ist zudem so niedrig, daß dieses Verfahren nicht zur technischen Herstellung von Cyanwasserstoff in Betracht gezogen werden kann.

Es ist auch bekannt, daß sich Ammoniumsalze organischer Carbonsäuren bei erhöhter Temperatur, etwa 300 °C, unter Bildung entsprechender Nitrile dehydratisieren lassen. Gemäß CS-Patent 160 810 (siehe Chem. Abstr. 85:80171) läßt sich in der in diesem Dokument beschriebenen Vorrichtung bei 300 °C Ammoniumoxalat in Dicyan und Ammoniumformiat in Cyanwasserstoff überführen. Zwar kommt bei dieser Umsetzung ein Ammoniumsalz zum Einsatz, jedoch handelt es sich nicht um eine Ammonoxidation, sondern um eine einfache Dehydratisierung.

Ammoniumsalze finden zwar verschiedentlich Anwendung bei der Herstellung von Ammonoxidationskatalysatoren, beispielsweise Ammoniummetavanadat gemäß US-Patent 4,447,612 und Ammoniumparamolybdat gemäß EP-A 0 107 638, jedoch ist der eigentliche Ammonoxidationskatalysator frei von Ammoniumsalzen, da der Herstellungsprozeß einen Temperprozeß bei 500 °C und darüber umfaßt.

Das Dokument Chemical Abstracts, AN 92:198128 (&JP-A 54145634) beschreibt die Herstellung einer aromatischen Cyanoverbindung mittels Ammonoxidation. Ein Gemisch von einem Alkohol, Sauerstoff (Luft), einer Ammoniumionen enthaltenden Verbindung und Metallkatalysator wird in einer wäßrigen Lösung auf 125 °C erhitzt. Beispiele der Ammoniumionen enthaltenden Verbindung sind z.B. die Salze, wie das Sulfat, Carbonat oder Chlorid.

Aufgabe der vorliegenden Erfindung ist ein Verfahren zur Herstellung von Cyanoverbindungen, insbesondere Cyanwasserstoff, gesättigte und ungesättigte alicyclische sowie aromatische und heteroaromatische Nitrile durch Ammonoxidation aufzuzeigen, bei welchem anstelle von gasförmigem Ammoniak eine andere Quelle hierfür eingesetzt werden kann.

Gelöst wird die Aufgabe durch ein Verfahren zur Herstellung von Cyanoverbindungen der allgemeinen Formel R-CN, worin R für H oder einen organischen Rest steht, durch Ammonoxidation einer ammonoxidierbaren organischen Verbindung mit einem Ammoniumsalz als Quelle für Ammoniak und einer Quelle für Sauerstoff in Gegenwart eines Ammonoxidationskatalysators, das dadurch gekennzeichnet ist, daß man in der Gasphase das Ammoniumsalz in Form einer Lösung oder eines feinen Pulvers, die ammonoxidierbare Verbindung gasförmig und die Quelle für Sauerstoff gleichzeitig mit dem Ammonoxidationskatalyator in Kontakt bringt und die Umsetzung bei 200 bis 700 °C durchführt.

Die Unteransprüche richten sich auf bevorzugte Ausführungsformen des erfindungsgemäßen Verfahrens.

Während bei der konventionellen Ammonoxidation eine gasförmige ammonoxidierbare organische Verbindung, gasförmiger Ammoniak und Luft als Quelle für Sauerstoff am Ammonoxidationskatalysator umgesetzt werden, wird im erfindungsgemäßen Verfahren als Quelle für Ammoniak ein Ammoniumsalz, vorzugsweise in Form einer wäßrigen oder organischen Lösung, eingesetzt. Die Lösung wird in feinstverteilter Form mit dem Ammonoxidationskatalysator in Kontakt gebracht; nach Verdampfen des Lösungsmittels findet in Gegenwart einer ammonoxidierbaren organischen Verbindung und Sauerstoff die Ammonoxidation an der Katalysatoroberfläche statt. Obgleich das Ammoniumsalz anstelle in gelöster Form auch in fein pulverisierter Form mit dem Amonoxidationskatalysator in Kontakt gebracht werden kann, wird die Verwendung einer Lösung des Ammoniumsalzes, insbesondere einer wäßrigen Lösung des Ammoniumsalzes bevorzugt, da hiermit eine feinere Verteilung und damit ein besserer Kontakt ermöglicht wird und eine höhere Ausbeute an ammonoxidierten Produkten, also den Cyanoverbindungen, erhältlich ist. Der Mechanismus der erfindungsgemäßen Ammonoxidation unter Verwendung von Ammoniumsalzen ist bisher nicht näher untersucht worden. Ob und in welchem Ausmaß der aus einer Zersetzung des Ammoniumsalzes gebildete Ammoniak oder das Ammoniumsalz an sich in die Ammonoxidation eingreift, ist damit noch nicht geklärt.

Es können sowohl anorganische als auch organische Ammoniumsalze zum Einsatz gelangen. Bevorzugt werden anorganische Ammoniumsalze, insbesondere Ammoniumsalze von Schwefelsäure, Schwefliger Säure, Phosphorsäure, Pyrophosphorsäure, Phosphoriger Säure, Salzsäure und Kohlensäure. Die Verwendbarkeit von Ammoniumsalzen der Schwefelsäure, Phosphorsäure und Salzsäure ist von besonderem technischen Interesse, da derartige Salze in vielen Prozessen als Nebenprodukt anfallen und entweder einer Entsorgung oder Wiederverwertung zugeführt werden müssen. Es ist somit ein besonderer Vorteil des erfindungsgemäßen Verfahrens, daß hiermit ein neuer Weg aufgezeigt wurde, derartige Ammoniumsalze zur Herstellung hochwertiger Cyanoverbindungen zu verwenden.

Die erfindungsgemäße Ammonoxidation läßt sich in üblichen Reaktoren, wie sie für Umsetzungen an Feststoffkatalysatoren Verwendung finden, durchführen. Der Katalysator kann beispielsweise in Form eines Festbetts in einem Festbettreaktor angeordnet sein oder in einem Wirbelschichtreaktor die Wirbelschicht bilden. Dem Reaktor werden gleichzeitig ein Ammoniumsalz, besonders bevorzugt eine wäßrige Lösung desselben, eine Quelle für Sauerstoff, üblicherweise Luft, sowie eine ammonoxidierbare organische Verbindung gasförmig zugeführt. Pro zu bildende Nitrilgruppe aus der ammonoxidierbaren Verbindung wird mindestens ein Äquivalent eines Ammoniumsalzes zugeführt; ein Überschuß im Bereich von 5 bis 100 %, insbesondere 10 bis 30 %, ist bei Ammonoxidationsreaktionen allgemein üblich. Die im Reaktionsraum anwesende Menge Sauerstoff muß mindestens jener Menge entsprechen, die gemäß der Formelgleichung der Ammonoxidation im konkreten Fall ermittelt wird.

Gemäß einer bevorzugten Ausführungsform erfolgt die Umsetzung in einem Wirbelschicht-Reaktor, wobei der Katalysator die Wirbelschicht bildet, ein Ammoniumsalz in Form einer wäßrigen Lösung auf die Wirbelschicht gesprüht wird und Luft sowie eine ammonoxidierbare Verbindung gasförmig durch die Wirbelschicht geleitet wird. Die optimale Konzentration der Ammoniumsalzlösung wird durch orientierende Vorversuche ermittelt.

Unter Verwendung oxidischer Ammonoxidationskatalysatoren erfolgt die Ammonoxidation bei einer Temperatur im Bereich von zwischen 200 und 700 °C und insbesondere zwischen 250 und 650 °C. Die optimale Reaktionstemperatur wird durch orientierende Vorversuche ermittelt. Eine derartige Ermittlung ist sehr zweckmäßig, da durch die Temperatur die Ausbeute an herzustellender Cyanoverbindung erheblich beeinflußt werden kann. Die optimale Temperatur wird durch die Art des Katalysators, die Reaktionspartner und den Reaktortyp beeinflußt. Wie aus den Beispielen folgt, wird zum Beispiel die Ammonoxidation zur Herstellung von HCN aus Methanol und Ammoniumsulfat unter Verwendung des beispielsgemäßen Katalysators vorzugsweise bei 350 bis 500 °C durchgeführt.

Dem erfindungsgemäßen Verfahren ist jede ammonoxidierbare organische Verbindung zugänglich, die auch im konventionellen Ammonoxidationsverfahren unter Einsatz von gasförmigem Ammoniak in eine Cyanoverbindung überführt werden kann. Eine wichtige Voraussetzung ammonoxidierbarer Verbindungen ist, daß sie selbst oder unter den Reaktionsbedingungen gezielt erzeugte Spaltprodukte derselben über eine ausreichende thermische Stabilität verfügen, so daß sie bei der gewählten Temperatur der Ammonoxidation nicht oder nur in geringem Umfang weiter zersetzt werden. Bevorzugte ammonoxidierbare Verbindungen enthalten mindestens eine Gruppe aus der Reihe Methyl, Vinyl, Vinyliden, Methylol (-CH₂OH) und Formyl (-CHO). Bei den ammonoxidierbaren Verbindungen kann es sich um alicyclische, cyclische, aromatische oder heteroaromatische Verbindungen mit einer oder mehreren der vorgenannten Gruppen handeln. Bevorzugte Verbindungen sind beispielsweise primäre Mono-, Di- und Polyalkohole mit 1 bis 12 C-Atomen, vorzugsweise 1 bis 6 C-Atomen, Mono- und Dialdehyde mit 1 bis 12 C-Atomen, vorzugsweise Monoaldehyde mit 1 bis 6 C-Atomen, geradkettige und verzweigte Alkane und Alkene mit jeweils 3 bis 12 C-Atomen, insbesondere 3 bis 6 C-Atomen, Mono-, Di- und Trialkylaromaten und -heteroaromaten, wobei die Alkylgruppe 1 bis 3 C-Atome, vorzugsweise 1 C-Atom enthält, und Ether oder Thioether mit mindestens einem aliphatischen C-Atom am Ethersauerstoff beziehungsweise Thioetherschwefel. Bei den zuvor genannten alkylierten Aromaten handelt es sich insbesondere um Benzol und Naphthalin mit einer oder mehreren Methylgruppen. Bei den alkylierten Heteroaromaten handelt es sich um solche, welche ein oder mehrere Heteroatome aus der Reihe N, O und S enthalten, 5 oder 6 Ringglieder aufweisen und ein oder zwei annelierte Ringe enthalten können. Beispiele bevorzugter alkylierter Heteroaromaten sind methylierte Pyridine, wie 2-, 3- und 4-Picolin, und 3-Methylthiophen. Bevorzugte alkylierte Aromaten sind insbesondere Toluol, ortho-, meta- und para-Xylol sowie 2-Methyl- und 1,4-Dimethyl-naphthalin. Ein Beispiel für einen ammonoxidierbaren Ether ist Methyl-tert.-butylether. Die erfindungsgemäße Ammonoxidation eignet sich in besonderer Weise zur Herstellung von Cyanwasserstoff unter Verwendung von Methanol oder Formaldehyd als ammonoxidierbarer Verbindung.

Die erfindungsgemäße Ammonoxidation läßt sich durch beliebige bekannte Ammonoxidationskatalysatoren katalysieren. Bei den üblichen Ammonoxidationskatalysatoren handelt es sich meistens um oxidische Katalysatoren mit zwei oder mehreren Metallen aus den Haupt- und Nebengruppen des Periodischen Systems der Elemente, ferner um Edelmetallkatalysatoren. Die Katalysatorkomponenten können als solche im Verfahren eingesetzt werden oder Bestandteil eines Trägerkatalysators sein, wobei der Träger insbesondere ausgewählt ist aus der Reihe von SiO₂, natürlichen und synthetischen Silikaten, Al₂O₃, TiO₂ und ZrO₂. Wirksame Katalysatorkomponenten sind zum Beispiel Phosphate, Vanadate, Molybdate und Wolframate der Elemente Mn, Fe, Co, Ni, Cu, Zn, Sb und Bi, wie binäre Metalloxide auf der Basis von V₂O₅-Sb₂O₅, V₂O₅-MoO₃, Bi₂O₃-MoO₃, Fe₂O₃-MoO₃, Fe₂O₃-WO₃, TiO₂-MoO₃ und ZrO₂-WO₃, welche zusätzlich bis zu 20 Atom-% ein oder mehrere andere Oxide von Zr, Te, Mo, W, V, Cr, Mn, Fe, Co, Ni, Zn, Sn, Bi und Sb enthalten können. Bei einer weiteren wirksamen Gruppe von Katalysatoren handelt es sich um Oxide der allgemeinen Formel MₐPOₓ, worin M ausgewählt ist aus der Reihe Mn, Fe, Co, Ni, Zn und B und a für eine beliebige Zahl zwischen 0,8 und 2 und x für die resultierende Zahl Sauerstoffatome zur Absättigung des Phosphats steht.

Der Katalysator kann pulverförmig eingesetzt werden, vielfach ist es jedoch zweckmäßiger, das Pulver in geeignete Formlinge zu überführen, um definierte Strömungsverhältnisse in einem Festbettreaktor zu erhalten oder Staubaustrag aus einem Wirbelschichtreaktor zu vermeiden. Es ist auch möglich, den Katalysator auf der Oberfläche von Kanälen eines monolithischen Katalysatorblocks zu fixieren.

Während der erfindungsgemäßen Umsetzung wird aus dem Ammoniumsalz die ihm zugrundeliegende Säure freigesetzt.

Sofern diese freigesetzte Säure unter den Reaktionsbedingungen nicht flüchtig ist, sondern sich auf dem Katalysator niederschlägt, kann es erforderlich sein, den Katalysator von Zeit zu Zeit durch eine entsprechende Wäsche zu regenerieren. Im Falle des Einsatzes eines Ammoniumsulfats wird gebildete Schwefelsäure als solche und/oder in Form von SO₃ mit dem Reaktionsgas aus dem Reaktor ausgetragen. Im Falle der Verwendung eines Ammoniumphosphats entstehen P₂O₅ und/oder andere Phosphoroxide, so daß ein damit beladener Katalysator regeneriert werden muß, beispielsweise durch Auswaschen des Katalysators mit Wasser, wobei als Wertstoff Phosphorsäure gewonnen wird.

Das den Reaktor verlassende gasförmige Reaktionsgemisch wird in an sich bekannter Weise aufgearbeitet. Die Aufarbeitung umfaßt im allgemeinen eine oder zwei Waschstufen, wobei einzelne Bestandteile des Reaktionsgasgemischs in der Waschflüssigkeit absorbiert und hieraus gewonnen werden können. Im Falle der Herstellung von Cyanwasserstoff unter Einsatz von Ammoniumsulfat kann beispielsweise ein erster Wäscher mit verdünnter Schwefelsäure der Absorption von SO₃ beziehungsweise H₂SO₄ dienen. Das den ersten Wäscher verlassende Reaktionsgas kann entweder durch einen mit Natrönlauge betriebenen Wäscher geleitet werden, wobei Cyanwasserstoff in Natriumcyanid überführt wird; gemäß einer alternativen Ausführungsform wird das Reaktionsgas aus dem ersten Wäscher in einer Gegenstromkolonne mit kaltem Wasser behandelt, wobei Cyanwasserstoff absorbiert wird und hieraus nach Stabilisierung durch Rektifizierung in flüssiger Form gewonnen werden kann. Bei der Herstellung von Nitrilen umfaßt die Aufarbeitung in der Regel eine Wäsche und/oder einen oder mehrere Destillations/Rektifikationsschritte.

Durch die Erfindung ist es erstmals möglich geworden, Ammoniumsalze, welche in vielen Prozessen als Nebenprodukte anfallen, als Quelle für Ammoniak anstelle des bisher üblichen gasförmigen Ammoniaks in der Ammonoxidation einzusetzen. Das Verfahren lehnt sich bezüglich des Ammonoxidationskatalysators und der Reaktionstemperatur eng an vorbekannte Verfahren zur Durchführung einer Ammonoxidation an, unterscheidet sich jedoch von diesen dadurch, daß das Ammoniumsalz in geeigneter Weise, vorzugsweise in Form einer wäßrigen Lösung, in den Reaktionsraum mit den übrigen Reaktionskomponenten und dem Katalysator in Kontakt gebracht wird.

Die Erfindung wird anhand der nachfolgenden Beispiele weiter erläutert.

### Beispiele

Als Ammonoxidationsreaktor diente ein Stahlrohr mit einem Durchmesser von 63 mm, das sich in einem elektrisch beheizten Ofen befand. In diesem Stahlrohr war eine 1 cm hohe Schüttung eines gemäß Beispiel 1 des US-Patents 4,447,612 hergestellten Ammonoxidationskatalysators auf der Basis von Sb₂O₃, V₂O₅, TiO₂, Montmorillonit und SiO₂ (BET-Oberfläche 200 m²/g) angeordnet. Die Katalysatorschicht wurde im Reaktionsrohr von Quarzwolle gehalten. Die Edukte, nämlich eine 40 gew.-%ige wäßrige Ammoniumsulfatlösung, Methanol und Luft wurden separat dem Reaktor zugeführt. Die Ammoniumsulatlösung wurde von oben mittels einer Einstoffdüse in den Reaktor eingespritzt, und zwar derart, daß möglichst keine Flüssigkeitströpfchen mit der Reaktorwand in Kontakt kamen. Methanol wurde in vorverdampfter Form oberhalb des Katalysators zugegeben und Luft über Kopf in den Reaktor eingeleitet. Das Reaktionsgas wurde am unteren Ende des Reaktors abgezogen. Die Reaktionsgase wurden zunächst durch einen Wäscher mit verdünnter Schwefelsäure und dann durch einen Wäscher mit Natronlauge geleitet. Nach Beendigung des Versuchs wurde der Cyanidgehalt in der Waschlösung des zweiten Wäschers trimetrisch bestimmt.

Aus Tabelle 1 folgen die Einsatzmengen, die Reaktionstemperatur (Ofen) und die Ausbeuten an HCN. Es ist festzustellen, daß die Reaktionsbedingungen nicht optimiert waren und jeweils zwischen 25 und 50 % des eingesetzten Ammoniumsulfats vor dem Katalysatorbett abgeschieden waren. Die in der Tabelle angegebenen Ausbeutewerte sind bezogen auf umgesetzte Stickstoffkomponenten (vor dem Katalysator auskristallisiertes Ammoniumsulfat wurde nicht in die Ausbeuteberechnung einbezogen). Selbst unter den sehr einfachen und in keiner Weise optimierten Bedingungen der durchgeführten Versuche war es überraschenderweise möglich, eine HCN-Ausbeute von über 50 %, bezogen auf umgesetzten Stickstoff, zu erhalten.

**Tabelle**

| Beispiel Nr. | 1 | 2 | 3 | 4 | 5 | 6 | 7 | 8 |
|---|---|---|---|---|---|---|---|---|
| Einspeisung: | | | | | | | | |
| Methanol (mMol/h) | 75 | 75 | 75 | 75 | 150 | 150 | 150 | 150 |
| (NH₄)₂SO₄ (mMol/h) | 55 | 55 | 55 | 55 | 110 | 110 | 110 | 110 |
| Luft (mMol O₂/h) | 650 | 650 | 650 | 650 | 1000 | 1000 | 1000 | 1000 |
| Ofentemp. (°C) | 550 | 350 | 370 | 390 | 410 | 430 | 450 | 470 |
| HCN-Ausbeute (%), bez. auf umgesetzten Stickstoff | 0,3 | 14 | 22 | 24 | 33 | 56 | 43 | 36 |

## Patentansprüche

1. Verfahren zur Herstellung von Cyanoverbindungen der allgemeinen Formel R-CN, worin R für H oder einen organischen Rest steht, durch Ammonoxidation einer ammonoxidierbaren organischen Verbindung mit einem Ammoniumsalz als Quelle für Ammoniak und einer Quelle für Sauerstoff in Gegenwart eines Ammonoxidationskatalysators,
dadurch gekennzeichnet,
daß man in der Gasphase das Ammoniumsalz in Form einer Lösung oder eines feinen Pulvers, die ammonoxidierbare Verbindung gasförmig und die Quelle für Sauerstoff gleichzeitig mit dem Ammonoxidationskatalyator in Kontakt bringt und die Umsetzung bei 200 bis 700 °C durchführt.

2. Verfahren nach Anspruch 1,
dadurch gekennzeichnet,
daß man als Ammoniumsalz ein Ammoniumsalz von Schwefelsäure, Phosphorsäure, Salzsäure oder Kohlensäure verwendet.

3. Verfahren nach Anspruch 1 oder 2,
dadurch gekennzeichnet,
daß man als ammonoxidierbare Verbindung eine solche aus der Reihe der primären Mono- und Dialkohole mit 1 bis 12 C-Atomen, der C₁- bis C₁₂ Mono- und Dialdehyde, der Alkane und Alkene mit jeweils 3 bis 12 C-Atomen, der C₁- bis C₃ mono- und dialkylierten Aromaten und Heteroaromaten und Ether mit mindestens einem aliphatischen C-Atom am Ethersauerstoff auswählt.

4. Verfahren nach einem der Ansprüche 1 bis 3,
dadurch gekennzeichnet,
daß man die Umsetzung bei 250 bis 650 °C durchführt.

5. Verfahren nach einem der Ansprüche 1 bis 4,
dadurch gekennzeichnet,
daß man das Ammoniumsalz in wäßriger oder organischer Lösung mit dem Ammonoxidationskatalysator in Kontakt bringt.

6. Verfahren nach einem der Ansprüche 1 bis 5,
dadurch gekennzeichnet,
daß man die Umsetzung in einem Wirbelschichtreaktor durchführt, wobei der Katalysator die Wirbelschicht bildet, eine wäßrige Ammoniumsalzlösung auf die Wirbelschicht sprüht und Luft und eine ammonoxidierbare Verbindung gasförmig durch die Wirbelschicht leitet.

7. Verfahren nach einem der Ansprüche 1 bis 6,
dadurch gekennzeichnet,
daß man als Ammonoxidationskatalysator einen solchen aus der Reihe (i) binärer Metalloxide auf der Basis V₂O₅-Sb₂O₅, V₂O₅-MoO₃, Bi₂O₃-MoO₃, Fe₂O₃-MoO₃, Fe₂O₃-WO₃, TeO₂-MoO₃ und ZrO₂-WO₃, welche zusätzlich bis zu 20 Atom-% ein oder mehrere andere Oxide von Zr, Te, Mo, W, V, Cr, Mn, Fe, Co, Ni, Zn, Sn, Bi und Sb enthalten können, (ii) Oxide der Formel MₐPOx, worin M ausgewählt ist aus der Reihe Mn, Fe, Co, Ni, Zn und B, a für eine beliebige Zahl zwischen 0,8 und 2 und x für die resultierende Zahl Sauerstoffatome steht, oder (iii) Edelmetalle verwendet, welche als solche oder auf einem oxidischen Trägermaterial zum Einsatz gelangen.

8. Verfahren nach einem der Ansprüche 1 bis 7,
dadurch gekennzeichnet,
daß die herzustellende Cyanoverbindung Cyanwasserstoff ist und als ammonoxidierbare organische Verbindung Methanol oder Formaldehyd eingesetzt wird.

9. Verfahren nach einem der Ansprüche 1 bis 7,
dadurch gekennzeichnet,
daß man als ammonoxidierbare Verbindung ein Methylpyridin einsetzt und hieraus das entsprechende Cyanopyridin erzeugt.

## Claims

1. Process for the preparation of cyano compounds of the general formula R-CN, wherein R represents H or an organic radical, by ammonoxidation of an ammonoxidizable organic compound with an ammonium salt as the source of ammonia and a source of oxygen in the presence of an ammonoxidation catalyst,
characterized in that
the ammonium salt in the form of a solution or a fine powder, the ammonoxidizable compound in gaseous form and the source of oxygen are simultaneously brought into contact with the ammonoxidation catalyst in the gas phase and the reaction is carried out at 200 to 700 °C.

2. Process according to claim 1,
characterized in that
an ammonium salt of sulfuric acid, phosphoric acid, hydrochloric acid or carbonic acid is used as the ammonium salt.

3. Process according to claim 1 or 2,
characterized in that as the ammonoxidizable compound a compound is selected from the series consisting of primary mono- and dialcohols having 1 to 12 C atoms, C₁- to C₁₂ mono- and dialdehydes, alkanes and alkenes having in each case 3 to 12 C atoms, C₁- to C₃ mono- and dialkylated aromatics and heteroaromatics and ether having at least one aliphatic C atom on the ether oxygen.

4. Process according to one of claims 1 to 3,
characterized in that
the reaction is carried out at 250 to 650 °C.

5. Process according to one of claims 1 to 4,
characterized in that
the ammonium salt is brought into contact with the ammonoxidation catalyst in aqueous or organic solution.

6. Process according to one of claims 1 to 5,
characterized in that
the reaction is carried out in a fluidized bed reactor, wherein the catalyst forms the fluidized bed, an aqueous ammonium salt solution is sprayed on to the fluidized bed, and air and an ammonoxidizable compound are passed through the fluidized bed in gaseous form.

7. Process according to one of claims 1 to 6,
characterized in that
the ammonoxidation catalyst used is one from the series consisting of (i) binary metal oxides based on V₂O₅-Sb₂O₅, V₂O₅-MoO₃, Bi₂O₃-MoO₃, Fe₂O₃-MoO₃, Fe₂O₃-WO₃, TeO₂-MoO₃ and ZrO₂-WO₃, which can additionally contain up to 20 atom% of one or more other oxides of Zr, Te, Mo, W, V, Cr, Mn, Fe, Co, Ni, Zn, Sn, Bi and Sb, (ii) oxides of the formula MₐPOx, wherein M is chosen from the series consisting of Mn, Fe, Co, Ni, Zn and B, a represents any desired number between 0.8 and 2 and x represents the resulting number of oxygen atoms, or (iii) noble metals, which are employed as such or on an oxidic support material.

8. Process according to one of claims 1 to 7,
characterized in that
the cyano compound to be prepared is hydrogen cyanide, and methanol or formaldehyde is employed as the ammonoxidizable organic compound.

9. Process according to one of claims 1 to 7,
characterized in that
a methylpyridine is employed as the ammonoxidizable compound and the corresponding cyanopyridine is produced therefrom.

## Revendications

1. Procédé de production de composés cyano de formule générale R-CN, dans laquelle R représente H ou un radical organique, par ammonoxydation d'un composé organique ammonoxydable avec un sel d'ammonium comme source d'ammoniac et une source d'oxygène en présence d'un catalyseur d'ammonoxydation,
caractérisé en ce que
dans la phase gazeuse on met en contact le sel d'ammonium sous la forme d'une solution ou d'une poudre fine, le composé ammonoxydable sous forme gazeuse et la source d'oxygène en même temps que le catalyseur d'ammonoxydation et on conduit la réaction entre 200 et 700°C.

2. Procédé selon la revendication 1,
caractérisé en ce qu'
on utilise comme sel d'ammonium un sel d'ammonium de l'acide sulfurique, de l'acide phosphorique, de l'acide chlorhydrique ou de l'acide carbonique.

3. Procédé selon la revendication 1 ou 2,
caractérisé en ce qu'
on utilise comme composé ammonoxydable un composé de la série des mono- et di-alcools primaires ayant de 1 à 12 atomes de carbone, des mono- et dialdéhydes en C₁ à C₁₂, des alcanes et alcènes ayant à chaque fois de 3 à 12 atomes de carbone, des composés aromatiques et hétéroaromatiques mono- et dialkylés en C₁ à C_{3,} ainsi que des éthers ayant au moins un atome de carbone aliphatique sur l'oxygène de l'éther.

4. Procédé selon l'une des revendications 1 à 3,
caractérisé en ce qu'
on conduit la réaction entre 250 et 650°C.

5. Procédé selon l'une des revendications 1 à 4,
caractérisé en ce qu'
on met le sel d'ammonium en solution aqueuse ou organique en contact avec le catalyseur d'ammonoxydation.

6. Procédé selon une des revendications 1 à 5,
caractérisé en ce qu'
- on conduit la réaction dans un réacteur à couche fluidisée, le catalyseur formant la couche fluidisée,
- on projette une solution aqueuse de sel d'ammonium sur la couche fluidisée ainsi que de l'air et un composé ammonoxydable sous forme gazeuse à travers la couche fluidisée.

7. Procédé selon l'une des revendications 1 à 6,
caractérisé en ce qu'
on utilise comme catalyseur d'ammonoxydation un catalyseur de la série (i) des oxydes métalliques binaires à base de V₂O₅-Sb₂O₅, V₂O₅-MoO₃, Bi₂O₃-MoO₃, Fe₂O₃-MoO₃, Fe₂O₃-WO₃, TeO₂-MoO₃ et ZrO₂-WO₃, qui peuvent contenir en outre jusqu'à 20 % atomique d'un ou plusieurs autres oxydes de Zr, Te, Mo, W, V, Cr, Mn, Fe, Co, Ni, Zn, Sn, Bi et Sb, (ii) des oxydes de formule MₐPOₓ, dans laquelle M est choisi dans la série Mn, Fe, Co, Ni, Zn et B, a représente un nombre quelconque choisi entre 0,8 et 2, et x représente le nombre résultant d'atomes d'oxygène, ou (iii) des métaux précieux, qui sont employés tels quels ou sur un support contenant des groupes oxydes.

8. Procédé selon l'une des revendications 1 à 7
caractérisé en ce que
le composé cyano à fabriquer est l'acide cyanhydrique et en ce qu'on utilise comme composé organique ammonoxydable le méthanol ou le formaldéhyde.

9. Procédé selon l'une des revendications 1 à 7,
caractérisé en ce qu'
on introduit comme composé ammonoxydable une méthylpyridine et en ce qu'on en obtient la cyanopyridine correspondante.
